# EUROPÄISCHE PATENTSCHRIFT

(11) **EP 1 725 415 B1**
(45) Veröffentlichungstag und Bekanntmachung des Hinweises auf die Patenterteilung: **09.04.2008**
(21) Anmeldenummer: 05706926.2
(22) Anmeldetag: 19.01.2005
(51) Int. Cl.: B60K 7/00

(54) **LENK-UND RADANTRIEB FÜR EIN FLURF ÖRDERZEUG**
STEERING AND DRIVING SYSTEM FOR AN INDUSTRIAL TRUCK
ENTRAINEMENT DE DIRECTION ET DE ROUE POUR CHARIOT DE MANUTENTION

(30) Priorität: 11.02.2004 DE 102004006722; 12.05.2004 DE 102004023341
(43) Veröffentlichungstag der Anmeldung: 29.11.2006
(73) Patentinhaber: ZF FRIEDRICHSHAFEN AG, 88038 Friedrichshafen (DE)
(72) Erfinder: SCHARFENBERG, Stephan, 99869 Tüttleben (DE); STREIPARDT, Peter, 99880 Waltershausen (DE)
(86) Internationale Anmeldenummer: PCT/EP2005/000485
(87) Internationale Veröffentlichungsnummer: WO 2005/077695

(56) Entgegenhaltungen:
- EP-A- 0 507 137
- DE-A1- 10 130 100
- DE-A1- 19 904 552
- DE-A1- 19 949 351

## Beschreibung

Die Erfindung betrifft einen Lenk- und Radantrieb für ein Flurförderzeug gemäß dem Oberbegriff des Patentanspruchs 1.

Aus der DE 41 10 792 C2 ist ein lenkbarer Radantrieb für ein Flurförderzeug mit zwei Antriebsrädern bekannt. Zwischen diesen ist ein Kegelradgetriebe mit zwei Kegelradsätzen angeordnet, deren jeweilige Ausgangswellen die Antriebsräder antreiben. Das Kegelradgetriebe wird von einem elektrischen Antriebsmotor über ein Planetengetriebe angetrieben, der parallel oder koaxial zur zentralen Drehachse der Vorrichtung angeordnet ist. Zudem ist ein Lenkantriebsmotor vorgesehen, der außerhalb des Radgetriebegehäuses direkt am Fahrgestell des Flurförderzeuges oder an einer zusätzlichen, dem Fahrgestell drehfest zugeordneten Kopfplatte befestigt ist. Bei einer Betätigung dieses Lenkantriebsmotors dreht dieser über eine Lenkgetriebestufe das Radgetriebegehäuse oder den Getriebeträger in seinem Drehkranzlager und damit auch das Radgetriebe mit den beiden Antriebsrädern um die Lenkachse. Nachteilig an diesem lenkbaren Radantrieb ist, dass die seitliche Anordnung zumindest des Lenkantriebsmotors die Gesamtvorrichtung wenig kompakt macht.

Außerdem zeigt die DE 199 04 552 A1 einen Radantrieb für ein Flurförderzeug, bei dem ein elektrischer Fahrmotor und ein von diesem angetriebenes Fahrgetriebe koaxial zur Längsachse der Radnabe eines Laufrades angeordnet sind. Der Fahrmotor ist dabei als Scheibenläufermotor ausgebildet. Zudem weist der Radantrieb eine elektromotorische Lenkung mit einem Lenkmotor und einem Lenkgetriebe auf, bei der der Lenkmotor im wesentlichen senkrecht zu einer Ebene ausgerichtet ist, die bei einem Drehen des Laufrades durch die gemeinsame Längsachse von Radnabe, Fahrmotor und Fahrgetriebe aufgespannt wird. Das Lenkgetriebe ist beispielsweise ein Wolfrom-Getriebe.

Wenngleich dieser Radantrieb durch den genannten Radnabenantrieb sowie die Integration des Lenkmotors und des Lenkgetriebes in die Gesamtvorrichtung sehr kompakt ist, so ist dessen konstruktiver Aufbau insbesondere wegen der beengten Verhältnisse im Bereich der Radnabe vergleichsweise komplex und daher kostenaufwendig. Zudem wird es als nachteilig erachtet, dass die offenbarte Kombination aus Fahrmotor und Fahrgetriebe sowie Lenkmotor und Lenkgetriebe einen relativ durchmessergroßen Radantrieb entstehen lässt.

Ein weiterer Radantrieb ist aus der DE 19949351 bekannt (beinhaltet die Merkmale des Oberbegriffs des Anspruchs 1)

Vor diesem Hintergrund liegt der Erfindung die Aufgabe zugrunde, einen konstruktiv einfachen elektromotorischen Lenk- und Fahrantrieb für ein Flurförderzeug zu schaffen, der bei akzeptabler Bauhöhe einen gegenüber bekannten Lenk- und Radantrieben kleinen Durchmesser aufweist und vergleichsweise kostengünstig herstellbar ist.

Die Lösung dieser Aufgabe ergibt sich aus den Merkmalen des Hauptanspruchs, während vorteilhafte Weiterbildungen und Ausgestaltungen der Erfindung den abhängigen Ansprüchen entnehmbar sind.

Die vorliegende Erfindung betrifft demnach einen Lenk- und Radantrieb für ein Flurförderzeug mit einem Fahrmotor, einem Fahrgetriebe, einem Lenkmotor und einem Lenkgetriebe, durch den wenigstens ein auf einer Radnabe des Flurförderzeuges angeordnetes Laufrad antreibbar und um eine Vertikallachse schwenkbar ist. Bei diesem Lenk- und Radantrieb ist nun von besonderer Bedeutung, dass der Fahrmotor, der Lenkmotor und das Lenkgetriebe koaxial zueinander angeordnet sind. Dadurch ergeben sich bei akzeptabler Bauhöhe insbesondere eine radial sehr kompakte Bauweise sowie durch den erhöhten Integrationsgrad, beispielsweise durch die Doppelnutzung von Gehäusebestandteilen, vergleichsweise geringe Herstellkosten.

Eine besonders bevorzugte Ausführungsform der Erfindung sieht dabei vor, dass der Fahrmotor, der Lenkmotor und das Lenkgetriebe axial eben in dieser Reihenfolge hintereinander angeordnet sind, während das als Winkelgetriebe ausgebildete Fahrgetriebe mit einem von dem Lenkmotor über das Lenkgetriebe angetriebenen Drehkranz verbunden und axial hinter dem Lenkgetriebe angeordnet ist.

In konstruktiver Weiterbildung des vorgenannten Bauprinzips ist vorgesehen, dass die Fahrmotorwelle als Vollwelle und die Lenkmotorwelle als Hohlwelle ausgebildet sowie die Fahrmotorwelle koaxial durch die Lenkmotorwelle geführt ist.

Zudem ist vorgesehen, dass die Fahrmotorwelle an ihrem von dem Fahrmotor fernen Ende ein Stirnrad trägt, welches mit einem Stirnrad auf der Eingangswelle des als Kegelradgetriebe ausgebildeten Fahrgetriebes im Zahneingriff steht. Die Ausgangswelle dieses Fahrgetriebes ist mit einer Radnabe des wenigstens einen Laufrades verbunden.

In weiterer Ausgestaltung der Erfindung ist das Lenkgetriebe als mehrstufiges Planetengetriebe beziehungsweise als Wolfrom-Getriebe ausgebildet, wobei letzteres wegen seiner axial geringen Länge bei hoher Untersetzung diesbezügliche Vorteile aufweist.

Zudem betrifft die Erfindung die antriebstechnische Verbindung zwischen dem Lenkmotor und dem Lenkgetriebe sowie zwischen dem Lenkgetriebe und dem Schwenkantrieb des Fahrgetriebes bzw. des mit letzterem verbundenen wenigstens einen Laufrades. In diesem Zusammenhang ist vorgesehen, dass die Lenkmotorwelle als ein erstes Sonnenrad ausgebildet ist, dessen Außenverzahnung mit den Zähnen der Planetenräder des Lenkgetriebes im Zahneingriff steht.

Bei der Nutzung eines mehrstufigen Planetengetriebes als Lenkgetriebe ist bevorzugt vorgesehen, dass die Planetenräder einer ersten Planetenradstufe mit dem genannten ersten Sonnenrad kämmen und auf einem ersten Planetenträger drehbar gelagert sind, welcher mit einem zweiten Sonnenrad drehfest verbunden ist. Auf diesem zweiten Planetenträger drehbar gelagerte Planetenräder einer zweiten Planetenradstufe kämmen zudem mit der Außenverzahnung des zweiten Sonnenrades, wobei sich die Planetenräder der ersten und der zweiten Planetenradstufe mit einem feststehenden Hohlrad oder zwei feststehenden Hohlrädern im Zahneingriff befinden. Außerdem ist vorgesehen, dass der zweite Planetenträger mit einem dritten Sonnenrad drehfest verbunden ist, dass das dritte Sonnenrad mit Planetenrädern einer dritten Planetenradstufe im Zahneingriff steht, dass die Planetenräder der dritten Planetenradstufe auf einem dritten Planetenträger drehbar gelagert sind, welcher drehfest mit dem Hohlrad verbunden ist, und dass die Planetenräder des dritten Planetenradsatzes mit einer Innenverzahnung eines Lagerinnenringes eines Drehkranzlagers im Zahneingriff stehen, welcher drehfest mit dem Drehkranz oder direkt mit dem Gehäuse des Fahrgetriebes verbunden ist.

Ein anderes Detail der Erfindung ist dadurch gekennzeichnet, dass der Lageraußenring des Drehkranzlagers mit einem Fahrzeugrahmen des Flurförderzeuges drehfest verbunden ist.

Zudem kann vorgesehen sein, dass das Gehäuse des Lenkmotors über Befestigungsmittel axial auf dem Lageraußenring gehalten wird. Des weiteren wird die kompakte Bauweise dieses Lenk- und Radantriebes dadurch begünstigt, dass zwischen der Außenwand des Lenkmotorgehäuses und dem Lageraußenring axial hintereinander das genannte Hohlrad und das radial äußere Ende des dritten Planetenträgers angeordnet sind. Auf diese Weise kann durch die genannten Befestigungsmittel das Lenkmotorgehäuse derart gegen den Lageraußenring des Drehkranzlagers festgelegt werden, dass gleichzeitig auch das als Lenkgetriebegehäuse wirkende Hohlrad sowie der dritte Planetenträger axial miteinander verbunden sind.

Es kann abweichend davon aber auch vorgesehen sein, dass das Lenkgetriebegehäuse über gesonderte Befestigungsmittel drehfest auf dem Lageraußenring des Drehkranzlagers befestigt ist.

Eine bevorzugte Variante der Erfindung ist dadurch gegeben, dass am fahrgetriebefernen Ende des Lenk- und Radantriebes eine auf die Fahrmotorwelle wirkende Bremse angeordnet ist. Diese Bremse ist bevorzugt als eine elektrisch betätigbare Bremse ausgebildet, während es hinsichtlich des Lenkmotors als sinnvoll erachtet wird, diesen als Scheibenläufermotor auszubilden.

Zudem kann vorgesehen sein, dass das Gehäuse des Fahrmotors an dem Gehäuse des Lenkmotors befestigt ist, oder von beiden Aggregaten ein gemeinsames Gehäuse genutzt wird. Dieses Gehäuse und/oder die Einzelgehäuse sind beispielsweise als Blechkonstruktionen aufgebaut.

Außerdem wird es als vorteilhaft beurteilt, wenn das Lenkgetriebegehäuse, der Lageraußenring des Drehkranzlagers oder der Drehkranz eine Bohrung zur Aufnahme eines Drehwinkelsensors aufweisen, mit deren Hilfe die Verschwenkung des Laufrades um seine Lenkachse ermittelbar und einem Steuergerät mitteilbar ist.

Hinsichtlich der Erfassung des Lenkwinkels zur Nutzung in einem dem Lenk- und Radantrieb zugeordneten Steuerungsgerät können am Rotor des Lenkmotors, an dem feststehenden Lageraußenring des Drehkranzlagers und/oder an dem Drehkranz Signalgeber wie Magnete vorgesehen sein, die mit den genannten Drehwinkelsensoren zur Drehwinkelerkennung zusammenwirken.

Nach einem anderen Aspekt der Erfindung ist vorgesehen, dass an dem Lenkgetriebegehäuse sowie an dem Lenkmotorgehäuse jeweils ein radial nach außen weisender Kragen, vorzugsweise Ringkragen, ausgebildet ist, durch deren jeweilige axiale Bohrungen Befestigungsschrauben zur Befestigung derselben an dem Lageraußenring geführt sind.

Ein derart mit einzelnen oder allen genannten Merkmalen ausgebildeter hochintegrierter Lenk- und Radantrieb weist als weitere Vorteile eine reduzierte Geräuschbelastung insbesondere bei Lastwechseln auf und ist wartungsarm bzw. wartungsfrei, weil das Lenkgetriebe mit einem Lebensdauerschmiermittel befüllbar ist, und auch der Lenkmotor durch Verwendung eines bürstenlosen Gleich- oder Wechselstrommotors in Standard- oder Scheibenläuferausführung wartungsfrei ausgebildet sein kann.

Zur Reduzierung der Herstellkosten wird des weiteren vorgeschlagen, die Planetenträger als Blechkonstruktionen in offener oder geschlossener Bauweise auszuführen.

Zur Verdeutlichung der Erfindung ist der Beschreibung eine Zeichnung beigefügt, in welcher drei Ausführungsformen des Lenk- und Radantriebes dargestellt sind. Im einzelnen zeigen
- Fig. 1: eine teilweise geschnitten dargestellte Seitenansicht eines elektromotorischen Lenk- und Radantriebes für ein Flurförderzeug, und
- Fig. 2: eine Darstellung wie in Fig. 1, jedoch bezüglich zweier weiterer Ausführungsbeispiele der Erfindung.

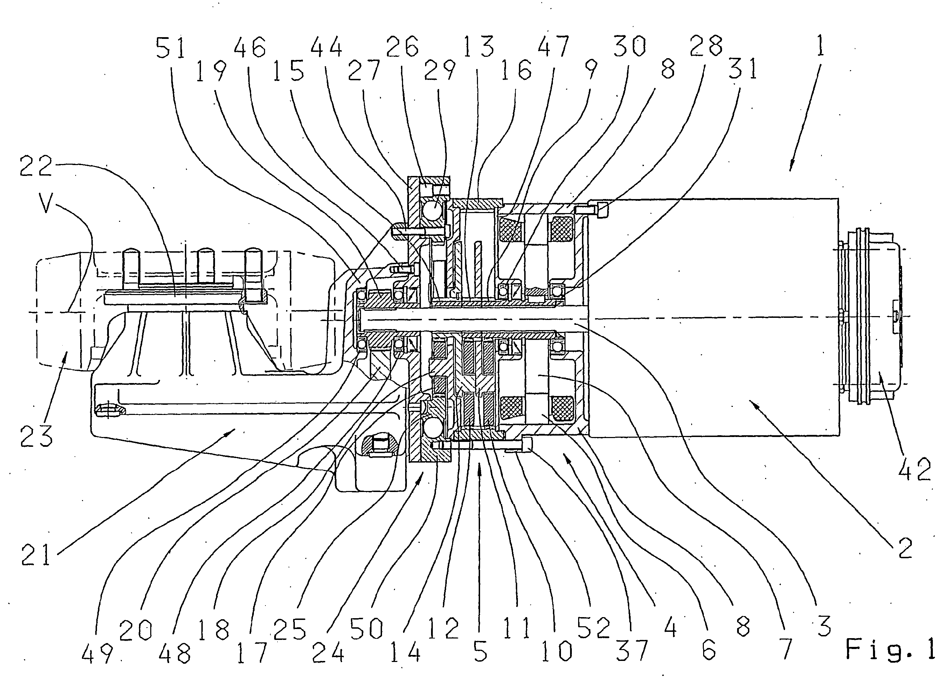

Gemäß Fig. 1 umfasst ein erfindungsgemäß ausgebildeter Lenk- und Radantrieb 1 für ein Flurförderzeug als Hauptkomponente zunächst einen Fahrmotor 2, über dessen Fahrmotorwelle 3 auf noch zu erläuternder Weise über ein Fahrgetriebe 21 ein Laufrad 23 angetrieben wird. Zum Steuern des Flurförderzeuges 1 ist das Laufrad 23 zusammen mit dem Fahrgetriebe 21 um eine Vertikalachse V schwenkbar. Zur Durchführung dieser Schwenkbewegung verfügt der Lenk- und Radantrieb 1 über einen elektrischen Lenkmotor 4, dem abtriebsseitig ein Lenkgetriebe 5 zur Untersetzung der Lenkmotordrehzahl zugeordnet ist. Der Ausgang dieses Lenkgetriebes 5 wirkt auf einen Drehkranz 27, der mit dem Gehäuse 51 des Fahrgetriebes 21 drehfest verbunden ist. Von besonderer Bedeutung bei dem erfindungsgemäß aufgebauten Lenk- und Radantrieb 1 ist, dass der Fahrmotor 2, der Lenkmotor 4 und das Lenkgetriebe 5 koaxial zueinander angeordnet sind.

Im Detail ist dieser Lenk- und Radantrieb 1 nun so aufgebaut, dass die Fahrmotorwelle 3 als Vollwelle ausgebildet und durch eine als Hohlwelle aufgebaute Lenkmotorwelle 9 geführt ist. An ihrem fahrmotorfernen Ende ist an der Fahrmotorwelle 3 ein Stirnrad 19 befestigt, welches mit einem Stirnrad 20 kämmt, das auf der nicht dargestellten Getriebeeingangswelle des Fahrgetriebes 21 sitzt. In diesem Fahrgetriebe wird das Antriebsmoment in an sich bekannter Weise über einen Winkeltrieb zu einer Getriebeausgangswelle geleitet, die mit der Radnabe 22 drehfest verbunden ist. Auf dieser Radnabe 22 ist das Laufrad 23 mittels Schrauben befestigt.

Zudem ist oberhalb des Fahrmotors 2 koaxial zu der Vertikalachse V eine Bremse 42 am Fahrmotorgehäuse befestigt, die bei Bedarf auf die Fahrmotorwelle 3 wirkt und vorzugsweise mit Federkraft betätigt und elektrisch gelüftet ist.

Der Lenkmotor 4 ist hier als Scheibenläufermotor ausgebildet und verfügt über einen Stator 6 und einen Rotor 7, die in einem Lenkmotorgehäuse 8, 8' untergebracht sind. Dabei ist der Stator 6 über nicht weiter gekennzeichnete Befestigungselemente mit dem Lenkmotorgehäuse 8, 8' verbunden, während der Rotor 7 an der bereits genannten Lenkmotorwelle 9 befestigt ist.

Wie in Fig. 1 links von der Vertikalachse V dargestellt ist, kann das Gehäuse des Fahrmotors 2 mittels Befestigungsschrauben 28 an dem Lenkmotorgehäuse 8 befestigt sein, während der rechten Zeichnungshälfte eine davon abweichende Bauform entnehmbar ist, bei der der Lenkmotor 4 und der Fahrmotor 2 ein gemeinsames Gehäuse 8' aufweisen.

Zudem zeigt Fig. 1, dass die fahrmotorferne Seite des Lenkmotors 4 von einem Deckel 47 abgedeckt ist, der zwischen dem Gehäuse 8, 8' des Lenkmotors 3 und einem auch als Gehäuseelement dienenden Hohlrad 16 axial eingeklemmt ist.

Die Lenkmotorwelle 9 ist über Wälzlager 30, 31 an dem als Lagerschild dienenden Deckel 47 und an dem Gehäuse 8, 8' des Lenk- und/oder Fahrmotors drehbar gelagert sowie über Dichtungen abgedichtet. Sie dient in einem dem Lenkmotor 4 antriebstechnisch nachgeordneten mehrstufigen Planeten-Lenkgetriebe 5 als erstes Sonnenrad. Die Außenverzahnung dieses ersten Sonnenrades 9 kämmt dabei mit den Zähnen von Planetenrädern 10 einer ersten Planetenradstufe, die auf einem ersten Planetenträger 11 drehbar gelagert sind. Dieser erste planetenträger 11 ist mit einem zweiten Sonnenrad 13 drehfest verbunden, welches sich axial in Richtung zum Stirnrad 19 an das erste Sonnenrad 9 (Lenkmotorwelle 9) anschließt.

Die Außenverzahnung dieses zweiten Sonnenrades 13 steht mit Zähnen von Planetenrädern 12 einer zweiten Planetenradstufe im Zahneingriff, die auf einem zweiten Planetenträger 14 drehbar gelagert sind. Dabei stehen die Planetenrädern 10, 12 der ersten und der zweiten Planetenradstufe mit dem feststehenden Hohlrad 16 im Zahneingriff.

Der zweite Planetenträger 14 ist mit einem dritten Sonnenrad 15 drehfest verbunden, welches mit Planetenrädern 17 einer dritten Planetenradstufe kämmt. Diese Planetenräder 17 der dritten Planetenradstufe sind auf einem dritten Planetenträger 18 drehbar gelagert, welcher drehfest mit dem genannten Hohlrad 16 verbunden ist.

Schließlich stehen die Planetenräder 18 des dritten Planetenradsatzes antriebswirksam mit einer Innenverzahnung eines Lagerinnenringes 25 eines Drehkranzlagers 24 im Zahneingriff, welcher über Befestigungsschrauben 44 drehfest mit dem Drehkranz 27 verbunden ist. Zu diesem Drehkranzlager 24 gehört ein Außenring 50 sowie zwischen diesen beiden Ringen 25, 50 eingeschlossene Wälzkörper 29.

Weiter ist in Fig. 1 erkennbar, dass vorzugsweise auch das Fahrgetriebegehäuse 51 über Befestigungsschrauben 46 mit dem Drehkranz 27 drehfest verbunden ist. Zudem verdeutlicht diese Darstellung, dass die Fahrmotorwelle 3 oberhalb des Stirnrades 19 über ein Wälzlager 48 im Drehkranz 26 und unterhalb desselben über ein Wälzlager 49 im Fahrgetriebegehäuse 51 drehgelagert ist.

Der Außenring 50 des Drehkranzlagers 24 ist mit dem hier nicht dargestellten Fahrgestell des Flurförderzeuges fest verbindbar, wozu in diesem Außenring 50 eine Bohrung 26 mit einem Schraubgewinde vorgesehen ist, in die eine diese beiden Teile verbindende Befestigungsschraube einschraubbar ist.

Schließlich zeigt die Fig. 1, dass am Gehäuse 8 des Lenkmotors 4 ein radial nach außen weisender Kragen 52, welcher auch als eine oder mehrere Laschen ausgebildet sei kann, angeordnet ist, durch dessen Axialbohrung eine Befestigungsschraube 37 geführt ist. Der Gewindeabschnitt dieser Befestigungsschraube 37 ist in ein Gewinde in dem Außenring 50 des Drehkranzlagers 24 eingeschraubt, so dass das Gehäuse 8' des Lenkmotors mit dem als Gehäuseabschnitt dienendem Hohlrad 16 und dem dritten Planetenträger 18 sowie Deckel 47 axial gegen den Lageraußenring 50 und damit auch gegen das Fahrgestell des Flurförderzeugs festgelegt sind.

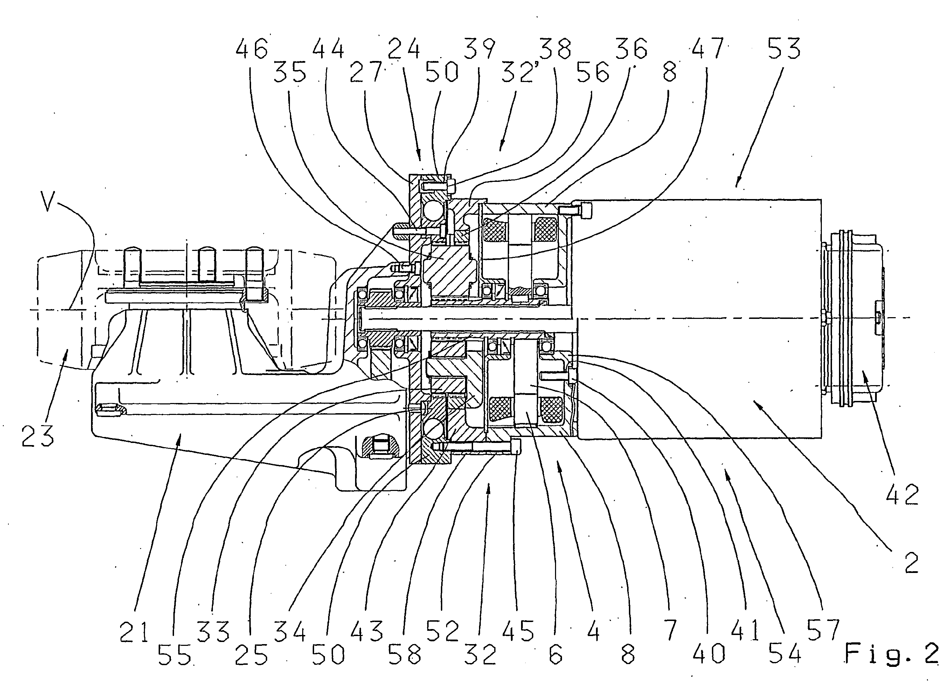

Der in Fig. 2 dargestellte Aufbau von zwei weiteren Varianten von erfindungsgemäß ausgebildeten Lenk- und Radantrieben 53, 54 unterscheidet sich von dem in Fig. 1 dargestellten Lenk- und Radantrieb 1 dadurch, dass das Lenkgetriebe hier nicht als mehrstufiges Planetengetriebe sondern als Wolfrom-Getriebe 32, 32' ausgebildet ist.

Auch bei diesen beiden Ausführungsformen dient die Lenkmotorwelle 55 als Sonnenrad und trägt demgemäss eine Außenverzahnung, die mit den Zähnen der Planetenräder 33 bzw. 35 im Zahneingriff stehen. Bei der rechts neben der Vertikalachse V dargestellten zweiten Bauvariante sind die Planetenräder 33 auf einem Planetenträger 34 drehgelagert und stehen zudem mit einer Innenverzahnung an einem radial nach innen weisenden Abschnitt des Lenkgetriebegehäuses 43 im Zahneingriff. Zudem kämmen die Planetenräder 33 mit der Innenverzahnung des schon aus Fig. 1 bekannten Innenringes 25 des Drehkranzlagers 24, so dass der Drehkranz 27 um die Vertikalachse V von dem Lenkmotor 4 drehbar ist.

Das Hohlrad 36 ist dabei an dem radial nach innen ragenden Abschnitt des Lenkgetriebegehäuses 43 befestigt oder mit ihm integriert, welches seinerseits mittels Befestigungsschrauben 45 am Außenring 50 des Drehkranzlagers 24 festgelegt ist.

Wie Fig. 2 rechts von der Vertikalachse V zeigt, ist es in diesem Zusammenhang vorteilhaft, wenn diese Befestigungsschrauben 45 Aufnahmeöffnungen in dem Kragen 52 des Lenkmotorgehäuses 8 sowie auch Bohrungen im Kragen 58 des Lenkgetriebegehäuses 43 durchdringt, so dass auf diese Weise die beiden oberen Antriebsaggregate 2, 4 zu dem Lenkgetriebegehäuse 43 zentriert und gegen den Lageraußenring 50 des Drehkranzlagers 24 festgelegt sind.

Bei der links von der Vertikalachse V dargestellten dritten Variante weisen die Planetenräder 35 des Wolfrom-Getriebes 32' eine andere Geometrie als die vorgenannten Planetenräder 33 auf. Dabei fällt besonders auf, dass diese keine Bohrung zur Aufnahme einer Drehachse eines Planetenträgers, sondern an den Planetenrädern 33 ausgebildete Drehachsen aufweisen. Diese greifen in Aufnahmeöffnungen eines hier nur andeutungsweise sichtbaren Planetenträgers ein.

Außerdem ist in Fig. 2 erkennbar, dass das axial sehr kurz bauende Lenkgetriebe 32' ein Hohlrad 36 aufweist, mit dem die Planeten 35 im Zahneingriff stehen. Dieses Hohlrad 36 ist mit einem radial nach innen weisenden Abschnitt des Lenkgetriebegehäuses 43 drehfest verbunden oder integriert. Das Lenkgetriebegehäuse 43 ist seinerseits unter Zwischenlage des Deckels bzw. Lagerschildes 47 des Lenkmotors 4 axial zwischen dessen Gehäuse 8 und dem Außenring 50 des Drehkranzlagers 24 festgeklemmt.

Schließlich zeigt Fig. 2, dass in den Lenk- und Radantrieb 32, 32' Sensoren 38, 40 vorteilhaft eingebaut werden können, mit denen die Drehung des Drehkranzes 27 gegen die darüber liegende Antriebsaggregate feststellbar ist.

Im Falle der zweiten Variante des Lenk- und Radantriebes 32 ist dieser Drehwinkelsensor 40 in eine Aufnahmeöffnung 41 eines radial nach innen weisenden und auch als Lagerschild dienenden Abschnitts 57 des gemeinsamen Gehäuses 8' von Fahr- und Lenkmotor eingesetzt und misst die genannte Drehbewegung an dem Rotor 7 des Lenkmotors 4.

Bei der dritten Variante des Lenk- und Radantriebes 32' (links neben der Vertikalachse V dargestellt) ist ein solcher Sensor 38 in eine Aufnahmeöffnung 39 im Außenring 50 des Drehkranzlagers 24 eingesetzt. In diesem Fall wird die Drehung zwischen dem Außenring 50 und dem Drehkranz 27 ermittelt und einem hier nicht dargestellten Steuerungsgerät mitgeteilt.

### Bezugszeichen

- 1: Lenk- und Radantrieb
- 2: Fahrmotor
- 3: Fahrmotorwelle
- 4: Lenkmotor
- 5: Lenkgetriebe
- 6: Stator Lenkmotor
- 7: Rotor Lenkmotor
- 8: Gehäuse Lenkmotor
- 9: Lenkmotorwelle; erstes Sonnenrad
- 10: Planetenrad der ersten Planetenradstufe
- 11: Erster Planetenträger
- 12: Planetenrad der zweiten Planetenradstufe
- 13: Zweites Sonnenrad
- 14: Zweiter Planenträger
- 15: Drittes Sonnenrad
- 16: Hohlrad
- 17: Planetenrad der dritten Planetenradstufe
- 18: Dritter Planetenträger
- 19: Stirnrad
- 20: Stirnrad
- 21: Fahrgetriebe
- 22: Radnabe
- 23: Laufrad
- 24: Drehkranzlager
- 25: Lagerinnenring mit Innenradverzahnung
- 26: Bohrung
- 27: Drehkranz
- 28: Befestigungsschraube
- 29: Wälzkörper
- 30: Wälzlager
- 31: Wälzlager
- 32: Wolfrom-Lenkgetriebe (Variante 2)
- 32': Wolfrom-Lenkgetriebe (Variante 3)
- 33: Planetenrad des Wolfrom-Lenkgetriebes (Variante 2)
- 34: Planetenträger des Wolfrom-Lenkgetriebes
- 35: Planetenrad des Wolfrom-Lenkgetriebes (Variante 3)
- 36: Hohlrad des Wolfrom-Lenkgetriebes
- 37: Befestigungsschraube
- 38: Drehwinkelsensor
- 39: Aufnahmeöffnung im Außenring des Drehkranzlagers
- 40: Drehwinkelsensor
- 41: Aufnahmeöffnung im Gehäuseabschnitt 57
- 42: Bremse
- 43: Gehäuse Wolfrom-Getriebe (Variante 2)
- 44: Befestigungsschraube
- 45: Befestigungsschraube
- 46: Befestigungsschraube
- 47: Deckel des Lenkgetriebes
- 48: Wälzlager
- 49: Wälzlager
- 50: Außenring des Drehkranzlagers
- 51: Gehäuse des Fahrgetriebes
- 52: Kragen am Gehäuse 8
- 53: Lenk- und Radantrieb
- 54: Lenk- und Radantrieb
- 55: Lenkmotorwelle für Wolfrom-Getriebe
- 57: Gehäuseabschnitt am Gehäuse 8'
- 58: Laschen oder Kragen am Lenkgetriebegehäuse 43

- V: Vertikalachse

## Patentansprüche

1. Lenk- und Radantrieb (1, 53, 54) für ein Flurförderzeug mit einem Fahrmotor (2), einem Fahrgetriebe (21), einem Lenkmotor (4) und einem Lenkgetriebe (5, 32, 32'), durch den wenigstens ein auf einer Radnabe (22) angeordnetes Laufrad (23) antreibbar und um eine Vertikallachse (V) schwenkbar ist, wobei der Fahrmotor (2), der Lenkmotor (4) und das Lenkgetriebe (5, 32, 32') koaxial zueinander angeordnet sind, **dadurch gekennzeichnet, dass** der Fahrmotor (2) über zwei Stimräder (19, 20) das Fahrgetriebe antreibt, und der Fahrmotor (2), der Lenkmotor (4) und das Lenkgetriebe (5, 31, 32') axial in dieser Reihenfolge hintereinander angeordnet sind und das Fahrgetriebe (21) mit einem von dem Lenkmotor (4) angetriebenen Drehkranz (27) verbunden ist und axial hinter dem Lenkgetriebe angeordnet ist.

2. Lenk- und Radantrieb nach Anspruch 1 oder 2, **dadurch gekennzeichnet, dass** eine Fahrmotorwelle (3) als Vollwelle und eine Lenkmotorwelle (9, 55) als Hohlwelle ausgebildet ist.

3. Lenk- und Radantrieb nach Anspruch 3, **dadurch gekennzeichnet, dass** die Fahrmotorwelle (3) koaxial durch die Lenkmotorwelle (9, 55) geführt ist.

4. Lenk- und Radantrieb nach einem der vorherigen Ansprüche, **dadurch gekennzeichnet, dass** die Fahrmotorwelle (3) an ihrem von dem Fahrmotor (2) fernen Ende ein Stirnrad (19) trägt, welches mit einem Stirnrad (20) auf der Eingangswelle des Fahrgetriebes (21) im Zahneingriff steht.

5. Lenk- und Radantrieb nach Anspruch 5, **dadurch gekennzeichnet, dass** das Stirnrad (20) auf der Eingangswelle des als Kegelradgetriebe ausgebildeten Fahrgetriebes (21) befestigt ist, dessen Ausgangswelle mit einer Radnabe (22) des wenigstens einen Laufrades (23) verbunden ist.

6. Lenk- und Radantrieb nach einem der vorherigen Ansprüche, **dadurch gekennzeichnet, dass** Lenkgetriebe (5) als mehrstufiges Planetengetriebe beziehungsweise als Wolfrom-Getriebe (32, 32') ausgebildet ist.

7. Lenk- und Radantrieb nach Anspruch 7, **dadurch gekennzeichnet, dass** die Lenkmotorwelle als ein erstes Sonnenrad (9, 55) ausgebildet ist, dessen Außenverzahnung mit den Zähnen der Planetenräder (10, 33, 35) des Lenkgetriebes (5, 32, 32') im Zahneingriff steht.

8. Lenk- und Radantrieb nach Anspruch 7 oder 8, **dadurch gekennzeichnet,**
**dass** Planetenräder (10) einer ersten Planetenradstufe mit dem ersten Sonnenrad (9) kämmen und auf einem ersten Planetenträger (11) drehbar gelagert sind, welcher mit einem zweiten Sonnenrad (13) drehfest verbunden ist,
**dass** auf einem zweiten Planetenträger (14) drehbargelagerte Planetenräder (12) einer zweiten Planetenradstufe mit der Außenverzahnung des zweiten Sonnenrades (13) kämmen,
**dass** die Planetenräder (11,12) der ersten und der zweiten Planetenradstufe mit einem feststehenden Hohlrad (16) im Zahneingriff stehen,
**dass** der zweite Planetenträger (14) mit einem dritten Sonnenrad (15) drehfest verbunden ist,
**dass** das dritte Sonnenrad (15) mit Planetenrädern (17) einer dritten Planetenradstufe im Zahneingriff steht,
**dass** die Planetenräder (17) der dritten Planetenradstufe auf einem dritten Planetenträger (18) drehbar gelagert sind, welcher drehfest mit dem Hohlrad (16) verbunden ist,
und **dass** die Planetenräder (17) der dritten Planetenradstufe mit einer Innenverzahnung eines Lagerinnenringes (25) eines Drehkranzlagers (24) im Zahneingriff stehen, welcher drehfest mit einem Drehkranz (27) oder direkt mit dem Gehäuse (51) des Fahrgetriebes (21) verbunden ist.

9. Lenk- und Radantrieb nach einem der vorherigen Ansprüche, **dadurch gekennzeichnet, dass** der Lageraußenring (50) des Drehkranzlagers (24) mit einem Fahrzeugrahmen des Flurförderzeuges drehfest verbunden ist.

10. Lenk- und Radantrieb nach einem der vorherigen Ansprüche, **dadurch gekennzeichnet, dass** das Gehäuse (8, 8') des Lenkmotors (4) über Befestigungsmittel (37) axial mit dem Lageraußenring (50) verbunden ist.

11. Lenk- und Radantrieb nach einem der vorherigen Ansprüche, **dadurch gekennzeichnet, dass** zwischen der Außenwand des Lenkmotorgehäuses (8, 8') und dem Lageraußenring (50) das Hohlrad (16) sowie das radial äußere Ende des dritten Planetenträgers (18) angeordnet sind.

12. Lenk- und Radantrieb nach einem der vorherigen Ansprüche, **dadurch gekennzeichnet, dass** das Lenkgetriebegehäuse (Hohlrad 16) über gesonderte Befestigungsmittel drehfest mit dem Lageraußenring (50) des Drehkranzlagers (24) verbunden ist.

13. Lenk- und Radantrieb nach einem der vorherigen Ansprüche, **dadurch gekennzeichnet, dass** am fahrgetriebefernen Ende des Lenk- und Radantriebes eine auf die Fahrmotorwelle (3) wirkende Bremse (42) angeordnet ist.

14. Lenk- und Radantrieb nach einem der vorherigen Ansprüche, **dadurch gekennzeichnet, dass** der Lenkmotor (4) als Scheibenläufermotor ausgebildet ist.

15. Lenk- und Radantrieb nach Anspruch 15, **dadurch gekennzeichnet, dass** das Gehäuse des Fahrmotors (2) an dem Gehäuse (8) des Lenkmotors (4) befestigt ist, oder dass beide Aggregate (2, 4) ein gemeinsames Gehäuse (8') nutzen.

16. Lenk- und Radantrieb nach einem der vorherigen Ansprüche, **dadurch gekennzeichnet, dass** das Lenkmotorgehäuse (8') eine Aufnahmeöffnung (41) zur Aufnahme eines Drehwinkelsensors (40) aufweist.

17. Lenk- und Radantrieb nach einem der vorherigen Ansprüche, **dadurch gekennzeichnet, dass** der Lageraußenring (50) des Drehkranzlagers (24) oder der Drehkranz (27) eine Aufnahmeöffnung (39) zur Aufnahme eines Drehwinkelsensors (38) aufweisen.

18. Lenk- und Radantrieb nach einem der vorherigen Ansprüche, **dadurch gekennzeichnet, dass** am Rotor (7) des Lenkmotors (4), an dem feststehenden Lageraußenring (50) des Drehkranzlagers (24) und/oder an dem Drehkranz (27) Signalgeber vorgesehen sein, die mit den genannten Drehwinkelsensoren zur Drehwinkelerkennung zusammenwirken.

19. Lenk- und Radantrieb nach einem der vorherigen Ansprüche, **dadurch gekennzeichnet, dass** an dem Gehäuse (43) des Lenkgetriebes (32, 32') sowie an dem Lenkmotorgehäuse (8, 8') jeweils ein radial nach außen weisender Kragen (52) oder Laschen (58) ausgebildet sind, durch deren jeweilige axiale Bohrungen Befestigungsschrauben (45) zur Befestigung derselben an dem Lageraußenring (50) geführt sind.

## Claims

1. Steering and wheel drive (1, 53, 54) for an industrial truck, comprising a travel motor (2), a travel transmission (21), a steering motor (4) and a steering transmission (5, 32, 32'), by means of which steering and wheel drive at least one travelling wheel (23), disposed on a wheel hub (22), can be driven and can be swivelled about a vertical axis (V), the travel motor (2), the steering motor (4) and the steering transmission (5, 32, 32') being disposed coaxially with each other, **characterized in that** the travel motor (2) drives the travel transmission via two spur gears (19, 20), and the travel motor (20), the steering motor (4) and the steering transmission (5, 31, 32') are disposed axially in series in this sequence, and the travel transmission (21) is connected to a slewing ring (27) driven by the steering motor (4) and is disposed axially behind the steering transmission.

2. Steering and wheel drive according to either of Claims 1 or 2, **characterized in that** a travel motor shaft (3) is realized as a solid shaft and a steering motor shaft (9, 55) is realized as a hollow shaft.

3. Steering and wheel drive according to Claim 3, **characterized in that** the travel motor shaft (3) is routed coaxially through the steering motor shaft (9, 55).

4. Steering and wheel drive according to any one of the preceding claims, **characterized in that** the travel motor shaft (3), at its end which is distant from the travel motor (2), carries a spur gear (19) which is in tooth engagement with a spur gear (20) on the input shaft of the travel transmission (21).

5. Steering and wheel drive according to Claim 5, **characterized in that** the spur gear (20) is fastened on the input shaft of the travel transmission (21), which is realized as a bevel-gear transmission and whose output shaft is connected to a wheel hub (22) of the at least one travelling wheel (23).

6. Steering and wheel drive according to any one of the preceding claims, **characterized in that** the steering transmission (5) is realized as a multistage planetary transmission or as a Wolfrom transmission (32, 32').

7. Steering and wheel drive according to Claim 7, **characterized in that** the steering motor shaft is realized as a first sun gear (9, 55), the outer toothing of which is in tooth engagement with the teeth of the planet gears (10, 33, 35) of the steering transmission (5, 32, 32').

8. Steering and wheel drive according to either of Claims 7 or 8, **characterized in that**
planet gears (10) of a first planet gear stage mesh with the first sun gear (9), and are rotatably mounted on a first planet carrier (11) that is connected to a second sun gear (13) in a rotationally rigid manner, planet gears (12) of a second planet gear stage that are rotatably mounted on a second planet carrier (14) mesh with the outer toothing of the second sun gear (13),
the planet gears (11, 12) of the first and the second planet gear stage are in tooth engagement with a stationary ring gear (16),
the second planet carrier (14) is connected to a third sun gear (15) in a rotationally rigid manner,
the third sun gear (15) is in tooth engagement with planet gears (17) of a third planet gear stage,
the planet gears (17) of the third planet gear stage are rotatably mounted on a third planet carrier (18) that is connected to the ring gear (16) in a rotationally rigid manner,
and the planet gears (17) of the third planet gear stage are in tooth engagement with an inner toothing of a bearing inner ring (25) of a slewing-ring bearing (24), which is connected to a slewing ring (27) in a rotationally rigid manner or is connected directly to the housing (51) of the travel transmission (21).

9. Steering and wheel drive according to any one of the preceding claims, **characterized in that** the bearing outer ring (50) of the slewing-ring bearing (24) is connected to a vehicle frame of the industrial truck in a rotationally rigid manner.

10. Steering and wheel drive according to any one of the preceding claims, **characterized in that** the housing (8, 8') of the steering motor (4) is connected axially to the bearing outer ring (50) via fastening means (37).

11. Steering and wheel drive according to any one of the preceding claims, **characterized in that** the ring gear (16) and the radially outer end of the third planet carrier (18) are disposed between the outer wall of the steering motor housing (8, 8') and the bearing outer ring (50).

12. Steering and wheel drive according to any one of the preceding claims, **characterized in that** the steering transmission housing (ring gear 16) is connected in a rotationally rigid manner, via separate fastening means, to the bearing outer ring (50) of the slewing-ring bearing (24).

13. Steering and wheel drive according to any one of the preceding claims, **characterized in that** a brake (42) which acts upon the travel motor shaft (3) is disposed at that end of the steering and wheel drive which is distant from the travel transmission.

14. Steering and wheel drive according to any one of the preceding claims, **characterized in that** the steering motor (4) is realized as a disc armature motor.

15. Steering and wheel drive according to Claim 15, **characterized in that** the housing of the travel motor (2) is fastened to the housing (8) of the steering motor (4), or both assemblies (2, 4) use a common housing (8').

16. Steering and wheel drive according to any one of the preceding claims, **characterized in that** the steering motor housing (8') has a receiving aperture (41) for accommodating a rotational angle sensor (40).

17. Steering and wheel drive according to any one of the preceding claims, **characterized in that** the bearing outer ring (50) of the slewing-ring bearing (24) or the slewing ring (27) has a receiving aperture (39) for accommodating a rotational angle sensor (38).

18. Steering and wheel drive according to any one of the preceding claims, **characterized in that** signal sources, which cooperate with the said rotational angle sensors for the purpose of rotational angle recognition, are provided on the rotor (7) of the steering motor (4), on the stationary bearing outer ring (50) of the slewing-ring bearing (24) and/or on the slewing ring (27).

19. Steering and wheel drive according to any one of the preceding claims, **characterized in that** a collar (52) or tabs (58), facing radially outwards, is/are realized respectively on the housing (43) of the steering transmission (32, 32') and on the steering motor housing (8, 8'), through the respective axial bores of which collars or tabs fastening screws (45) are routed, for the purpose of fastening the collars or tabs to the bearing outer ring (50).

## Revendications

1. Entraînement de direction et de roue (1, 53, 54) pour un chariot de manutention comprenant un moteur de propulsion (2), un mécanisme de propulsion (21), un moteur de direction (4) et un mécanisme de direction (5, 32, 32') par lesquels au moins une roue motrice (23) montée sur un moyeu de roue (22) peut être entraînée et être orientée autour d'un axe vertical (V), dans lequel le moteur de propulsion (2), le moteur de direction (4) et le mécanisme de direction (5, 32, 32') sont disposés coaxialement entre eux, **caractérisé en ce que** le moteur de propulsion (2) entraîne le mécanisme de propulsion par l'intermédiaire de deux roues droites (19, 20), le moteur de propulsion (2), le moteur de direction (4) et le mécanisme de direction (5, 32, 32') sont disposés l'un à la suite de l'autre dans la direction axiale, dans cet ordre de succession, et le mécanisme de propulsion (21) est relié à une couronne tournante (27) entraînée par le moteur de direction (4) et est disposé axialement en aval du mécanisme de direction.

2. Entraînement de direction et de roue selon la revendication 1, **caractérisé en ce qu'**un arbre (3) du moteur de propulsion est constitué par un arbre plein, et un arbre (9, 55) du moteur de direction est constitué par un arbre creux.

3. Entraînement de direction et de roue selon la revendication 2, **caractérisé en ce que** l'arbre (3) du moteur de propulsion est inséré coaxialement à travers l'arbre (9, 55) du moteur de direction.

4. Entraînement de direction et de roue selon l'une des revendications précédentes, **caractérisé en ce que** l'arbre (3) du moteur de propulsion porte à son extrémité éloignée du moteur de propulsion (2) une roue droite (19) qui est en prise par engrènement avec une roue droite (20) montée sur l'arbre d'entrée du mécanisme de propulsion (21).

5. Entraînement de direction et de roue selon la revendication 4, **caractérisé en ce que** la roue droite (20) est fixée sur l'arbre d'entrée du mécanisme de propulsion (21) constitué par un mécanisme à roues coniques et dont l'arbre de sortie est relié à un moyeu de roue (22) de l'au moins une roue motrice (23).

6. Entraînement de direction et de roue selon l'une des revendications précédentes, **caractérisé en ce que** le mécanisme de direction (5) est constitué par un mécanisme épicycloïdal à plusieurs étages ou par un mécanisme de Wolfrom (32, 32').

7. Entraînement de direction et de roue selon la revendication 6, **caractérisé en ce que** l'arbre du moteur de direction est constitué par une première roue planétaire (9, 55) dont la denture extérieure est en prise par engrènement avec les dents des roues satellites (10, 33, 35) du mécanisme de direction (5, 32, 32').

8. Entraînement de direction et de roue selon la revendication 6 ou 7, **caractérisé**
**en ce que** des roues satellites (10) d'un premier étage épicycloïdal engrènent avec la première roue planétaire (9) et sont montées rotatives sur un premier porte-satellites (11) qui est relié solidairement en rotation à une deuxième roue planétaire (13),
**en ce que** des roues satellites (12) d'un deuxième étage de roues satellites qui sont montées rotatives sur un deuxième porte-satellites (14) engrènent avec la denture extérieure de la deuxième roue planétaire (13),
**en ce que** les roues satellites (11, 12) des premier et deuxième étages de roues satellites sont en prise par engrènement avec une couronne à denture intérieure fixe (16),
**en ce que** le deuxième porte-satellites (14) est relié solidairement en rotation à une troisième roue planétaire (15),
**en ce que** la troisième roue planétaire (15) est en prise par engrènement avec des roues satellites (17) d'un troisième étage de roues satellites,
**en ce que** les roues satellites (17) du troisième étage de roues satellites sont montées rotatives sur un troisième porte-satellites (18) qui est relié solidairement en rotation à la couronne à denture intérieure (16),
et **en ce que** les roues satellites (17) du troisième étage de roues satellites sont en prise par engrènement avec une denture intérieure d'une bague intérieure (25) d'un palier de couronne tournante (24), laquelle est reliée solidairement en rotation à une couronne tournante (27) ou est reliée directement au carter (51) du mécanisme de propulsion (21).

9. Entraînement de direction et de roue selon l'une des revendications précédentes, **caractérisé en ce que** la bague extérieure (50) du palier de couronne tournante (24) est reliée solidairement en rotation à un châssis de véhicule du chariot de manutention.

10. Entraînement de direction et de roue selon l'une des revendications précédentes, **caractérisé en ce que** le carter (8, 8') du moteur de direction (4) est relié axialement à la bague extérieure (50) du palier par l'intermédiaire de moyens de fixation (37).

11. Entraînement de direction et de roue selon l'une des revendications précédentes, **caractérisé en ce que** la couronne à denture intérieure (16) ainsi que l'extrémité radialement extérieure du troisième porte-satellites (18) sont disposés entre la paroi extérieure du carter de moteur de direction (8, 8') et la bague extérieure (50) du palier.

12. Entraînement de direction et de roue selon l'une des revendications précédentes, **caractérisé en ce que** le carter du mécanisme de direction (la couronne à denture intérieure 16) est relié solidairement en rotation à la bague extérieure (50) du palier de couronne tournante (24) par l'intermédiaire de moyens de fixation distincts.

13. Entraînement de direction et de roue selon l'une des revendications précédentes, **caractérisé en ce qu'**un frein (42) agissant sur l'arbre (3) du moteur de propulsion est monté à l'extrémité de l'entraînement de direction et de roue qui est éloignée du mécanisme de propulsion.

14. Entraînement de direction et de roue selon l'une des revendications précédentes, **caractérisé en ce que** le moteur de direction (4) est constitué par un moteur à entrefer plat.

15. Entraînement de direction et de roue selon la revendication 14, **caractérisé en ce que** le carter du moteur de propulsion (2) est fixé au carter (8) du moteur de direction (4, ou **en ce que** les deux groupes (2, 4) utilisent un carter commun (8').

16. Entraînement de direction et de roue selon l'une des revendications précédentes, **caractérisé en ce que** le carter (8') du moteur de direction comporte une ouverture de réception (41) destinée à recevoir un capteur d'angle de rotation (40).

17. Entraînement de direction et de roue selon l'une des revendications précédentes, **caractérisé en ce que** la bague extérieure (50) du palier de couronne tournante (24) ou la couronne tournante (27) comporte une ouverture de réception (39) destinée à recevoir un capteur d'angle de rotation (38).

18. Entraînement de direction et de roue selon l'une des revendications précédentes, **caractérisé en ce que**, sur le rotor (7) du moteur de direction (4), sur la bague extérieure fixe (50) du palier de couronne tournante (24) et/ou sur la couronne tournante (27) sont prévus des émetteurs de signaux qui coopèrent avec les capteurs d'angle de rotation précités pour la détection d'un angle de rotation.

19. Entraînement de direction et de roue selon l'une des revendications précédentes, **caractérisé en ce que**, sur la carter (43) du mécanisme de direction (32, 32') et sur le carter (8, 8') du moteur de direction sont agencés respectivement un collet (52) ou des pattes (58) pointant radialement vers l'extérieur et à travers des perçages axiaux respectifs desquels sont enfilées des vis de fixation (45) servant à les fixer à la bague extérieure (50) du palier.
